# EUROPEAN PATENT SPECIFICATION

(11) **EP 1 969 124 B1**
(45) Date of publication and mention of the grant of the patent: **13.04.2016**
(21) Application number: 07701934.7
(22) Date of filing: 04.01.2007
(51) Int. Cl.: C12N 15/09, C12N 15/29, C12N 15/82, A01H 1/00, A01H 5/00, A01P 21/00

(54) **A RICE GENE, GS3, EXERTING PRIMARY CONTROL OVER GRAIN LENGTH AND GRAIN WEIGHT**
REISGEN (GS3) MIT KAPAZITÄT ZUR AUSÜBUNG DER PRIMÄREN KONTROLLE ÜBER KORNLÄNGE UND -GEWICHT
GENE DU RIZ APPELE GS3 REGULANT PRINCIPALEMENT LA LONGUEUR DU GRAIN ET LE POIDS DU GRAIN

(30) Priority: 05.01.2006 CN 200610018107
(43) Date of publication of application: 17.09.2008
(73) Proprietor: Huazhong Agricultural University, Hongshan District Wuhan, Hubei 430-070 (CN)
(72) Inventor: ZHANG, Qifa, Hubei 430070 (CN); FAN, Chuchuan, Hubei 430070 (CN); XING, Yongzhong, Hubei 430070 (CN)
(74) Representative: von Kreisler Selting Werner - Partnerschaft von Patentanwälten und Rechtsanwälten mbB
(86) International application number: PCT/CN2007/000002
(87) International publication number: WO 2007/076727

(56) References cited:
- WO-A1-2005/070195
- WO-A1-2005/094562
- WO-A2-2005/097824
- DATABASE EMBL [Online] 24 October 2002 (2002-10-24), "Oryza sativa chromosome 3 BAC OSJNBa0030J19 genomic sequence, complete sequence." XP002581506 retrieved from EBI accession no. EMBL:AC135559 Database accession no. AC135559
- TAN Y F ET AL: "Genetic bases of appearance quality of rice grains in Shanyou 63, an elite rice hybrid" THEORETICAL AND APPLIED GENETICS, SPRINGER, BERLIN, DE LNKD- DOI:10.1007/S001220051549, vol. 101, no. 5-6, 1 October 2000 (2000-10-01), pages 823-829, XP002227768 ISSN: 0040-5752
- XING YONG-ZHONG ET AL: "Mapping and isolation of quantitative trait loci controlling plant height and heading date in rice" ACTA BOTANICA SINICA, vol. 43, no. 7, July 2001 (2001-07), pages 721-726, XP9133466 ISSN: 0577-7496
- MCCOUCH S R ET AL: "QTL mapping in rice" TRENDS IN GENETICS, ELSEVIER SCIENCE PUBLISHERS B.V. AMSTERDAM, NL LNKD- DOI:10.1016/S0168-9525(00)89157-X, vol. 11, no. 12, 1 December 1995 (1995-12-01), pages 482-487, XP004207261 ISSN: 0168-9525
- CHUCHUAN FAN ET AL: "GS3, a major QTL for grain length and weight and minor QTL for grain width and thickness in rice, encodes a putative transmembrane protein" THEORETICAL AND APPLIED GENETICS ; INTERNATIONAL JOURNAL OF PLANT BREEDING RESEARCH, SPRINGER, BERLIN, DE LNKD- DOI:10.1007/S00122-006-0218-1, vol. 112, no. 6, 1 April 2006 (2006-04-01) , pages 1164-1171, XP019322238 ISSN: 1432-2242
- DATABASE GENBANK [Online] CHUCHUAN F. ET AL.: 'GS3, a major QTL for grain length and weight and minor QLT for grain width and thickness in rice, encodes a putative transmembrane protein', XP003015373 Database accession no. (DQ355996) & TAG THEORETICAL AND APPLIED GENETICS vol. 112, no. 6, 30 April 2006, pages 1164 - 1171
- DATABASE EMBL [Online] 24 October 2002 'Oryza sativa chromosome 3 BAC OSJNBa0030J19 genomic sequence, sequence version archive' Retrieved from EBI, accession no. EMBL:AC135559 Database accession no. AC135559
- DATABASE EMBL [Online] 16 December 2005 'Oryza sativa chromosome 3 BAC OSJNBa0030J19 genomic sequence, complete sequence.' Retrieved from EBI, accession no. EMBL:AC135559 Database accession no. AC135559
- DATABASE EMBL [Online] 16 April 2006 'Oryza sativa chromosome 3 BAC OSJNBa0030J19 genomic sequence, complete sequence.' Retrieved from EBI, accession no. EMBL:AC135559 Database accession no. AC135559
- DATABASE EMBL [Online] 16 January 2006 'Oryza sativa chromosome 3 BAC OSJNBa0030J19 genomic sequence, complete sequence, with hand notes' Retrieved from EBI, accession no. EMBL:AC135559 Database accession no. AC135559

### Sequence Listing

<110> Huazhong Agricultural University
<120> A rice gene, *GS3*, exerting primary control over grain length and grain weight
<130>
<140> PCT/CN07/000002
   <141> 2007-01-04
<150> CN 20061018107.4
   <151> 2006-01-05
<160> 35
<170> PatentIn version 3.1
<210> 1
   <211> 7883
   <212> DNA
   <213> Rice (Oryza sativa)
<220>
   <221> gene
   <222> (1)..(7883)
   <223>
<220>
   <221> 3'UTR
   <222> (7009)..(7222)
   <223>
<220>
   <221> exon
   <222> (6579)..(7008)
   <223>
<220>
   <221> exon
   <222> (4854)..(4907)
   <223>
<220>
   <221> exon
   <222> (4726)..(4770)
   <223>
<220>
   <221> exon
   <222> (3235)..(3287)
   <223>
<220>
   <221> exon
   <222> (1646)..(1762)
   <223>
<220>
   <221> 5'UTR
   <222> (1609)..(1645)
   <223>
<400> 1
   gccgccatga gctcaccggt gctgccgccg acctcctgcc tacgtgccgc cttctccgct     60
   gcaagtcgcc gctgctgccc tccccacgac acagctggat ccgggagggg agggcaggga    120
   aggttcgctg tcgccgccgc caccctccct acgacgtagc cagatatggg tggagaggta    180
   agcgccgccg ccgctgcaca acccacgatg ccgccgctgc acaacccgcg atgccgtcgg    240
   ccggatttgg gaggtggagc gtcgccgccc tccgccccgc gccatgtctg aggaaagaga    300
   gcgatgggag cgccgccggc caccaccgtc cccccttccc cctccaacca gatctgggag    360
   gaagggaggg agggagggga gccatcacgt cgttgggagt agatgccgcc accgccctcc    420
   ccctccctgc ggccaccaca ctggggtgca ccgccaccgc taggtagccg cctctggctg    480
   gatccacgcc gaggagagga aggagaggga gagggagagg gaggggaggg gagatgtgga    540
   agttgactta gaaaattttg acgcccggtg gtttttaaga tactttacct ttttgcaggc    600
   ggatttatta aagaggtccg tctgcaaaaa taatgatatt ttcacggtcg gacctcttaa    660
   gatatctaca tgtagaaatc tatatttttt tacttaaatt actgccaggg ctttgcctcg    720
   tagtataatt ttcataaaaa ctcaaaatat ttacaaatta caaaagagga aggaaggaga    780
   aaaacttata aagattacaa tgtttgtaac caatcaaaaa ctatctgctt taaaggatct    840
   ttcattctac acatatgaag aagaacttct tttctaaacg atattcttca tgaaccaaag    900
   gagggaagct cattccgaaa atttttccca tttctctgct cgtaggcgga taactatctc    960
   atctcacggt tgttattttt gtaggtggct atttggcttc cagagaccta ctcgtccggg   1020
   aaaaagaaat gccagcatct agaaaaatag tttttctagt agagtgttgt gagcgtctgc   1080
   gatcgtggta aagaaagcaa aaaaggtgaa ggacgagtgg catgatacat atgggaaagc   1140
   tgtagacttt gacccttgac tactcgttgg aagtgtgcgt ctgcatgcat tattgaacgg   1200
   ctctgatccc cgcggcgcag cggatcgggg tcatgtccgg atgggcatat cgacgagaag   1260
   gatccgtccc cgacaatctt tcaaggcccg tgcccccgtc cctcctctcc tctgcgcctt   1320
   tccatcatca tttacgccca accccaacac atgtacattt cccttggctt gcttccggag   1380
   aagaaaagag cggccatcca ctccactctc cactctctcc cttccatcat tacttgccca   1440
   aaaacggcaa tcccctcccc tccatctcca tgtgctcttc cacctagctc cgccattcaa   1500
   agcaaagcac caagcttttg cctctccctc taccatgcct gccccctata catagctgct   1560
   gcaccgtctc tcttcataaa tatactagta ggagtagcag agctcatcac ttcgatcatc   1620
   tccattatcg gaacttcgga gtgac atg gca atg gcg gcg gcg ccc cgg ccc     1672
                               Met Ala Met Ala Ala Ala Pro Arg Pro
                                1               5
   aag tcg ccg ccg gcg ccg ccc gac cca tgc ggc cgc cac cgc ctc cag     1720
   Lys Ser Pro Pro Ala Pro Pro Asp Pro Cys Gly Arg His Arg Leu Gln
    10                  15                  20                  25
   ctc gcc gtc gac gcg ctc cac cgc gag atc gga ttc ctc gag             1762
   Leu Ala Val Asp Ala Leu His Arg Glu Ile Gly Phe Leu Glu
                    30                  35
   gtacaatcta tctctatctg tctatatcac taccattcat actccttcga tcttgcttca   1822
   aaacaaaaaa atatatattt cctacttcat attcatatac acacgtacgg cttgctatct   1882
   gtcgaattgt ttgcttctgc atgcatgcat cactctcatt gtaagttttt cccagcttaa   1942
   aaccactcct tttatcttcg ttcttcttcc ttcttgtttt tttttaaaaa aacaacaact   2002
   catttaatct tcatatagtg tatcatgcat catttgcttc tttgatcagt tccccaaaaa   2062
   ctgctcctct cttcccagcc aaataattaa acttaagcaa acaagcaagt tgaactgatg   2122
   atccaataaa acaaaacaaa accgatcgaa taggaagtca atggcatata ccagctgcta   2182
   tagctgaagc cacgaatgct tagcttagct ctagtcgatc cctgttgact gttcaacaca   2242
   ctgcactaac acaccagtta atgagctgat taattaaacc attaaatgag cttaacgggc   2302
   ggctagcttc ttcctctggg cccgtgccga tcgtaccatc ggtttgcgcg tccctccacc   2362
   taaactctct gccttgttat tccctttgcc tagtactaca tgcatttgca tcatcatccc   2422
   atcaccaata gtactacgtt tcaactggat tttggtggtg tccaaccata tcatatttgg   2482
   ttttgttctc tagtttactc ctacattagt ctctagcggt tttgtggagt actaaagaaa   2542
   acaactaatc agccagggtt taacgtttaa tcggttggtg gttttgttaa ttaatttcat   2602
   ctactatttt agacttcaca ggtcttcgag ctataagcat cgattgccat gcatcaatcg   2662
   atgctggtcc acgctagttt ctgagttctg actagctctc ttaattgtgc tttgacctac   2722
   tttaattaat taaccagtgg ctgcgtcact cattgaccaa cattgtcatg ttacccggac   2782
   tgattttttt tttctttaaa aaaacaccgg atatattatt agttagtgta tatatatgtc   2842
   tgctcaagaa gcgcatgcat atagtttctc gtcaaacaaa aaatgtactg tatgctcaaa   2902
   gcatctgttt tggaattgtc atattcgcct ttataattaa aataattaaa atggtgatgc   2962
   ccagcttttt ttttcctcca ataatttatt tattggcttg atttcctgtg ctattaggag   3022
   taaaactact ccgttttaat tagcaccatt tttaaagctt ctaaaattaa cctaagtaaa   3082
   gtacgacagt acttgctgtc tagctttaaa tgttttgggt gttaaaatat ccctcagaca   3142
   tcacctgaaa agttgacagg ctaaacacat gcccatctcc ctcgtttact taaattaatt   3202
   cgaacaaaca actgtatata tatttcttgc ag ggt gaa ata aat tca atc gaa     3255
                                       Gly Glu Ile Asn Ser Ile Glu
                                        40                  45
   ggg atc cac gct gcc tcc aga tgc tgc aga ga  gtaagccagc ctgctgtttc   3307
   Gly Ile His Ala Ala Ser Arg Cys Cys Arg Glu
                50                  55
   tttttgtact acttccattt cttctcgtct ttactcttac catgcattca caaaatatac   3367
   ttacttaccc cagtttttga tcatgaactt tgaccgttat ctttttaagc aacttcaata   3427
   aaataggttt aaatgcaaat gttatgtaca ccattgatta taaaaccttg gcaaatgaaa   3487
   gtaaaaaacc agcacattta atttctgaac gttgggagta ttattatttt tatatctttt   3547
   actatcattt aatcatagta tcgtgcaagc tttttgagtg taattaggtt gcttaaggta   3607
   aaaaaatgta actaggttac atttagtact aaactgaaca tttaattagt aatgtttcgt   3667
   taagtaactg taatttcaat gcatgcatgt cctcccgtaa gagcaagttt aatagtatag   3727
   ccaactacta gctccaattt atttatagac aatctaatag ctcattcata caataattac   3787
   atactacact attaatatct gatcccacct gtcatacaca tactgcattt tggagtccgt   3847
   gctatagctg actacaaatc tatagtccgc tgctcttctc tctctttatt tatctcctta   3907
   aaatatgttt gcagctggct tatagcctgc tattgtacct gctctgaaaa tagtgcagag   3967
   actgttcaaa aagtcattgc acaataacta ttcacatgga actgtgaaaa gtatatattg   4027
   gaacttacta gctagatcct tttgggaaca tgggaaaagc caagtcacgt gtggaatccc   4087
   tattccctgt gttcttcagc tagaagagtg aaaataatgt actactacta tacggagatg   4147
   aaattacagc aggagcagaa agcgggaaaa aaactttaaa tcaattaaac aaacctctct   4207
   ctgcaaaatt caacaccagc aacgaacaac tcatcaagtt cttgtgttat gtaccggccg   4267
   gtaactaatt gttgtttgca taagcgaaac ggtatatttg caaacaaaaa ataatttatg   4327
   aataaaactt ttatatacat gttcttaatt atctcaaaac aaaggttgaa aaataaactt   4387
   cgatgaaaaa atctcaaaat caattccaaa tttatggtga aaattttaaa ttttgtctga   4447
   taaacataag tataagcaaa aaaaaaagta aagcaatgtc actatgttaa tggtattggt   4507
   atatatacct ttgagtttct gtctgtactt taggagtaca tgctacgaac atgttttctt   4567
   ggcttctatt tcgttggaat tacttgcgta ttgtgggcca gacgcctggt gaacttcgtc   4627
   gattgtgtgg actaattaag ctcacctgaa ataagtggtt gaaaacaagg ctaaagatga   4687
   ttttaatcat ggattttggc ttggaaattt ttttgtag g gtt gac gaa ttc atc    4741
                                             Val Asp Glu Phe Ile
                                                       60
   gga aga act cct gat cca ttc ata acg at  gtatggattt tcaggtcgag     4790
   Gly Arg Thr Pro Asp Pro Phe Ile Thr Ile
            65                  70
   aatttgtctt taacttcgca cgactgttat tttttttctt attaattctc tgtttacaag   4850
   cag t tca tcg gag aag cga agt cat gat cat tct cac cac ttc ttg aag   4899
         Ser Ser Glu Lys Arg Ser His Asp His Ser His His Phe Leu Lys
                 75                  80                  85
   aag ttt cg  gtactcactt cattcccgga tcttaatgta tatatgcata          4947
   Lys Phe Arg
            90
   tctgcactgt gctaattggt gtacacatta tgtgatcatc agtccaagtt aattattact   5007
   tacaaaactg aactaataaa cactagaaaa tatgtaactt gcaaagtaca tattgaatca   5067
   gggattcata tatagaactc cacctgcaga tttcttccaa tatatatatg ctgtcaccat   5127
   gttttcactt gtcacctagt acacctttga ctgggagact ttccttgatg atcgacgtgg   5187
   tcatattctt cagattgatt taatttcaga tagaaaaaaa tattgtttac ttagtttctc   5247
   tccttcagta agagagatgt gcaagaccag cgatcaaact atatgaactg ttcgtttcat   5307
   gataaaaaaa acatgatatg gaataactag gtgattcaac atataatggc tgataatccc   5367
   tcgtttcagg agataccatc agttttctac ttttctactt ttctccatgt tctctttttc   5427
   atgtttgagg tggatcggag cttgtattag atgtttgctc agctcaatta ttgctgcaga   5487
   tttccctata tagcctccac tgtatatata ctccctccga ttccataatt taatgatatt   5547
   ttgaacaatg acgctgtctc caaaatatat ctttcacttt gttttcctat tataatatat   5607
   acaataaaaa aaatacatat ttacttttct tataaatagt ttcaaagaca aatctatata   5667
   tgttgttata taactctttt aaactaaata tttttaaagt tatagtcaaa gttacaaaag   5727
   ttggacctca aacatgtata aaacgtcgag aattgtatat ctgatcaacc aataatttgt   5787
   agtgcattgt cttaaaaaaa ttgcatttct agctatgtat ctagataatt acaagaacca   5847
   ggtgaaatca cattttattt ttactggacc acgaactcat tgtttaatta cttccagcct   5907
   tgcactaaat aacaataatt gaacctggca tcacctgcac aattaatttg gacacaaagt   5967
   aaacatgaat gcaacatact tcgtttcata ttgtttgttg gtgtaggatt taaattttgt   6027
   gtcaaaatac ttgccgttct tactacattc ttaagcactt tgagaactaa ccttctcttc   6087
   ctacccttca tcaatacagt catactaact aattggctct tatgccttga aaaactaatt   6147
   aggatgtatt taatgagggt aaccatgtaa tctgccacca gtgaatgcaa ttttggttca   6207
   aaatttcggg cccccgccct aaaaagtcat tatctcgata gaattttttt gaatttagtc   6267
   aaaatttatt caaatttagc caaattgtgt taaatttcaa ataatttcag tctaaaaagt   6327
   gctgaaaatc ccgaaattta ggttctaccg aaatggccgg aaattttcag cgaaaatcaa   6387
   acatgaaaac cttgtctgcc acatttgtta gtttgtgtaa agatttgcta gaacgataag   6447
   taatttgaag cggatgtata ttatatatcc cacaaaacca tcaacttgtt aattacatgt   6507
   atatttgtgc atgatgcttt caccactttg tggttgttaa cgattaaatc acacgtttta   6567
   ttttctcaca g c tgt ttg tgc aga gca agt gcg tgc tgc ctc agc tac     6615
                  Cys Leu Cys Arg Ala Ser Ala Cys Cys Leu Ser Tyr
                                      95                  100
   ctc tcc tgg atc tgc tgc tgc agc agc gcc gcc ggc ggc tgc tca tcc    6663
   Leu Ser Trp Ile Cys Cys Cys Ser Ser Ala Ala Gly Gly Cys Ser Ser
           105                 110                 115
   tcc tcc tcc tcc tcc ttc aac ctc aag agg ccg agc tgc tgc tgc aac    6711
   Ser Ser Ser Ser Ser Phe Asn Leu Lys Arg Pro Ser Cys Cys Cys Asn
       120                 125                 130
   tgc aac tgc aac tgc tgc tcc tcc tcc tcc tcc tca tgt ggg gcg gcg    6759
   Cys Asn Cys Asn Cys Cys Ser Ser Ser Ser Ser Ser Cys Gly Ala Ala
   135                 140                 145                 150
   tta acg aag agt ccg tgt cgc tgc cgc cgc cgc agc tgc tgc tgc cgt    6807
   Leu Thr Lys Ser Pro Cys Arg Cys Arg Arg Arg Ser Cys Cys Cys Arg
                   155                 160                 165
   cgc tgc tgc tgc ggc ggc gtc ggc gtc cgc gcg tgc gcg agc tgc agc    6855
   Arg Cys Cys Cys Gly Gly Val Gly Val Arg Ala Cys Ala Ser Cys Ser
               170                 175                 180
   tgc tcc ccg ccg tgc gcg tgc tgc gcg ccg ccg tgc gcg gga tgc tcg    6903
   Cys Ser Pro Pro Cys Ala Cys Cys Ala Pro Pro Cys Ala Gly Cys Ser
           185                 190                 195
   tgc cgc tgc acc tgc ccg tgc ccg tgc ccc ggc ggc tgc tcc tgc gcg    6951
   Cys Arg Cys Thr Cys Pro Cys Pro Cys Pro Gly Gly Cys Ser Cys Ala
       200                 205                 210
   tgc ccg gcg tgc agg tgc tgc tgc ggc gtc cct cgt tgc tgc ccc ccc    6999
   Cys Pro Ala Cys Arg Cys Cys Cys Gly Val Pro Arg Cys Cys Pro Pro
   215                 220                 225                 230
   tgc ttg tga tcgatcgatc gattgagcga agctgcactg attggttaat          7048
   Cys Leu
   taattagttc tcgatgatga tcgatcgagc tgcgcgcgta cttaattagc tagctaggtt   7108
   ctggtgttaa ttagttcctc atcgatgcat atgttgattg ccttgctctg cttgcggtta   7168
   tctgtaattt ggctttgctg ccatgatgag tacgtgattg ctgatttatt ttacatatcc   7228
   tctgctatat atatctagct ggtagtagct agttttgatc tcacttgcaa aactattcat   7288
   ttcctgattt taacaaggtc aaatgacttg gttgcctttg gttcatactc cttagagcaa   7348
   gtataaaggt ttatatggag gagagaggta acaaaaaaaa atcaagagta ttggctctca   7408
   tgcgagagct agcttatcac aagctacaaa tcaaatatat taaatgtata agtaagagat   7468
   agagagagga ataaaattat agctaacctt ataggtaatg tattatatat gttaatttta   7528
   aaataagcta atagtaaaaa gtgagcttta ttattatcct tgctcttagt aatggcctcg   7588
   attcctacat tttttttttg agagagtagt acatattttc gcagcgtaat atggacattg   7648
   gccgacgggc ctaactatta ctgggtctaa gtgcctaact gagctaaaag ccttaaaccc   7708
   aatactgaaa acgaaaaagt tcactccggg tccctcatct tgtcgacggg ttttaaaatc   7768
   atcattgaac cggaatacgc attcccaatc tttcaaaacc ggatcacact aggtctaagg   7828
   cagtattgct cccgattttg gctgatatgg tggttgagtc agcgtgagac acaca       7883
<210> 2
   <211> 699
   <212> DNA
   <213> Rice (Oryza sativa)
<220>
   <221> CDS
   <222> (1) .. (699)
   <223>
<400> 2
   atg gca atg gcg gcg gcg ccc cgg ccc aag tcg ccg ccg gcg ccg ccc      48
   Met Ala Met Ala Ala Ala Pro Arg Pro Lys Ser Pro Pro Ala Pro Pro
    1               5                  10                  15
   gac cca tgc ggc cgc cac cgc ctc cag ctc gcc gtc gac gcg ctc cac      96
   Asp Pro Cys Gly Arg His Arg Leu Gln Leu Ala Val Asp Ala Leu His
               20                  25                  30
   cgc gag atc gga ttc ctc gag ggt gaa ata aat tca atc gaa ggg atc     144
   Arg Glu Ile Gly Phe Leu Glu Gly Glu Ile Asn Ser Ile Glu Gly Ile
           35                  40                  45
   cac gct gcc tcc aga tgc tgc aga gag gtt gac gaa ttc atc gga aga     192
   His Ala Ala Ser Arg Cys Cys Arg Glu Val Asp Glu Phe Ile Gly Arg
       50                  55                  60
   act cct gat cca ttc ata acg att tca tcg gag aag cga agt cat gat     240
   Thr Pro Asp Pro Phe Ile Thr Ile Ser Ser Glu Lys Arg Ser His Asp
    65                  70                  75                  80
   cat tct cac cac ttc ttg aag aag ttt cgc tgt ttg tgc aga gca agt     288
   His Ser His His Phe Leu Lys Lys Phe Arg Cys Leu Cys Arg Ala Ser
                   85                  90                  95
   gcg tgc tgc ctc agc tac ctc tcc tgg atc tgc tgc tgc agc agc gcc     336
   Ala Cys Cys Leu Ser Tyr Leu Ser Trp Ile Cys Cys Cys Ser Ser Ala
               100                 105                 110
   gcc ggc ggc tgc tca tcc tcc tcc tcc tcc tcc ttc aac ctc aag agg     384
   Ala Gly Gly Cys Ser Ser Ser Ser Ser Ser Ser Phe Asn Leu Lys Arg
           115                 120                 125
   ccg agc tgc tgc tgc aac tgc aac tgc aac tgc tgc tcc tcc tcc tcc     432
   Pro Ser Cys Cys Cys Asn Cys Asn Cys Asn Cys Cys Ser Ser Ser Ser
       130                 135                 140
   tcc tca tgt ggg gcg gcg tta acg aag agt ccg tgt cgc tgc cgc cgc     480
   Ser Ser Cys Gly Ala Ala Leu Thr Lys Ser Pro Cys Arg Cys Arg Arg
   145                 150                 155                 160
   cgc agc tgc tgc tgc cgt cgc tgc tgc tgc ggc ggc gtc ggc gtc cgc     528
   Arg Ser Cys Cys Cys Arg Arg Cys Cys Cys Gly Gly Val Gly Val Arg
                   165                 170                 175
   gcg tgc gcg agc tgc agc tgc tcc ccg ccg tgc gcg tgc tgc gcg ccg     576
   Ala Cys Ala Ser Cys Ser Cys Ser Pro Pro Cys Ala Cys Cys Ala Pro
               180                 185                 190
   ccg tgc gcg gga tgc tcg tgc cgc tgc acc tgc ccg tgc ccg tgc ccc     624
   Pro Cys Ala Gly Cys Ser Cys Arg Cys Thr Cys Pro Cys Pro Cys Pro
           195                 200                 205
   ggc ggc tgc tcc tgc gcg tgc ccg gcg tgc agg tgc tgc tgc ggc gtc     672
   Gly Gly Cys Ser Cys Ala Cys Pro Ala Cys Arg Cys Cys Cys Gly Val
       210                 215                 220
   cct cgt tgc tgc ccc ccc tgc ttg tga                             699
   Pro Arg Cys Cys Pro Pro Cys Leu
   225                 230
<210> 3
   <211> 232
   <212> PRT
   <213> Rice (Oryza sativa)
<400> 3
   Met Ala Met Ala Ala Ala Pro Arg Pro Lys Ser Pro Pro Ala Pro Pro
   1               5                   10                  15
   Asp Pro Cys Gly Arg His Arg Leu Gln Leu Ala Val Asp Ala Leu His
               20                  25                  30
   Arg Glu Ile Gly Phe Leu Glu Gly Glu Ile Asn Ser Ile Glu Gly Ile
           35                  40                  45
   His Ala Ala Ser Arg Cys Cys Arg Glu Val Asp Glu Phe Ile Gly Arg
       50                  55                  60
   Thr Pro Asp Pro Phe Ile Thr Ile Ser Ser Glu Lys Arg Ser His Asp
   65                  70                  75                  80
   His Ser His His Phe Leu Lys Lys Phe Arg Cys Leu Cys Arg Ala Ser
                   85                  90                  95
   Ala Cys Cys Leu Ser Tyr Leu Ser Trp Ile Cys Cys Cys Ser Ser Ala
               100                 105                 110
   Ala Gly Gly Cys Ser Ser Ser Ser Ser Ser Ser Phe Asn Leu Lys Arg
           115                 120                 125
   Pro Ser Cys Cys Cys Asn Cys Asn Cys Asn Cys Cys Ser Ser Ser Ser
       130                 135                 140
   Ser Ser Cys Gly Ala Ala Leu Thr Lys Ser Pro Cys Arg Cys Arg Arg
   145                 150                 155                 160
   Arg Ser Cys Cys Cys Arg Arg Cys Cys Cys Gly Gly Val Gly Val Arg
                   165                 170                 175
   Ala Cys Ala Ser Cys Ser Cys Ser Pro Pro Cys Ala Cys Cys Ala Pro
               180                 185                 190
   Pro Cys Ala Gly Cys Ser Cys Arg Cys Thr Cys Pro Cys Pro Cys Pro
           195                 200                 205
   Gly Gly Cys Ser Cys Ala Cys Pro Ala Cys Arg Cys Cys Cys Gly Val
       210                 215                 220
   Pro Arg Cys Cys Pro Pro Cys Leu
   225                 230
<210> 4
   <211> 20
   <212> DNA
   <213> Artificial sequence
   <223> Primer
<400> 4
   AGCAAAGCTG GAACGAAGAG 20
<210> 5
   <211> 20
   <212> DNA
   <213> Artificial sequence
   <223> Primer
<400> 5
   TAAATTACGC CGTGTCAACG 20
<210> 6
   <211> 20
   <212> DNA
   <213> Artificial sequence
   <223> Primer
<400> 6
   GCAACCAAGT CCACGCTAAT 20
<210> 7
   <211> 20
   <212> DNA
   <213> Artificial sequence
   <223> Primer
<400> 7
   TAGCCGAAGA TCAGCCTCCT 20
<210> 8
   <211> 20
   <212> DNA
   <213> Artificial sequence
   <223> Primer
<400> 8
   GATTATTGGA GACGGGACGA 20
<210> 9
   <211> 20
   <212> DNA
   <213> Artificial sequence
   <223> Primer
<400> 9
   GACGGCATGA CCACTCTTTT 20
<210> 10
   <211> 20
   <212> DNA
   <213> Artificial sequence
   <223> Primer
<400> 10
   AGCTTTGGTG TCGTTCTGCT 20
<210> 11
   <211> 20
   <212> DNA
   <213> Artificial sequence
   <223> Primer
<400> 11
   CCGACTTGGA GAGAATGGAA 20
<210> 12
   <211> 20
   <212> DNA
   <213> Artificial sequence
   <223> Primer
<400> 12
   GCTGTGTTGT CCTTTGCTGA 20
<210> 13
   <211> 20
   <212> DNA
   <213> Artificial sequence
   <223> Primer
<400> 13
   CCAATAAACC CCACTGCAAC 20
<210> 14
   <211> 20
   <212> DNA
   <213> Artificial sequence
   <223> Primer
<400> 14
   CTTTACAAAA CCGGCGGTAA 20
<210> 15
   <211> 20
   <212> DNA
   <213> Artificial sequence
   <223> Primer
<400> 15
   TGAAGCGGAC CTAGCATTTT 20
<210> 16
   <211> 20
   <212> DNA
   <213> Artificial sequence
   <223> Primer
<400> 16
   AAGAACGACT ACGCGCATCT 20
<210> 17
   <211> 20
   <212> DNA
   <213> Artificial sequence
   <223> Primer
<400> 17
   CCATCGCTCT CTTTCCTCAG 20
<210> 18
   <211> 20
   <212> DNA
   <213> Artificial sequence
   <223> Primer
<400> 18
   CAACACCAGC AACGAACAAC 20
<210> 19
   <211> 20
   <212> DNA
   <213> Artificial sequence
   <223> Primer
<400> 19
   ACGAGGGATT ATCAGCCATT 20
<210> 20
   <211> 20
   <212> DNA
   <213> Artificial sequence
   <223> Primer
<400> 20
   CGGTATGCCA AGTTGAATGA 20
<210> 21
   <211> 20
   <212> DNA
   <213> Artificial sequence
   <223> Primer
<400> 21
   TTGCCGCAGT AAACAAGAAG 20
<210> 22
   <211> 20
   <212> DNA
   <213> Artificial sequence
   <223> Primer
<400> 22
   CCTTCAGTAA GAGAGATGTG 20
<210> 23
   <211> 20
   <212> DNA
   <213> Artificial sequence
   <223> Primer
<400> 23
   AGTTGATGGT TTTGTGGGAT 20
<210> 24
   <211> 20
   <212> DNA
   <213> Artificial sequence
   <223> Primer
<400> 24
   TCTGCTTGCG GTTATCTGTA 20
<210> 25
   <211> 20
   <212> DNA
   <213> Artificial sequence
   <223> Primer
<400> 25
   TTAGGTCCCT TTTCTCGTCC 20
<210> 26
   <211> 20
   <212> DNA
   <213> Artificial sequence
   <223> Primer
<400> 26
   GTCTGAGGAA AGAGAGCGAT 20
<210> 27
   <211> 20
   <212> DNA
   <213> Artificial sequence
   <223> Primer
<400> 27
   AAGCAAGCCA AGGGAAATGT 20
<210> 28
   <211> 20
   <212> DNA
   <213> Artificial sequence
   <223> Primer
<400> 28
   AGCAAAAAAG GTGAAGGACG 20
<210> 29
   <211> 20
   <212> DNA
   <213> Artificial sequence
   <223> Primer
<400> 29
   CAAAGGGAAT AACAAGGCAG 20
<210> 30
   <211> 19
   <212> DNA
   <213> Artificial sequence
   <223> Primer
<400> 30
   CGAATAGGAA GTCAATGGC 19
<210> 31
   <211> 20
   <212> DNA
   <213> Artificial sequence
   <223> Primer
<400> 31
   GTCGTACCCG CCTTAGTTGA 20
<210> 32
   <211> 20
   <212> DNA
   <213> Artificial sequence
   <223> Primer
<400> 32
   TGCCCATCTC CCTCGTTTAC 20
<210> 33
   <211> 18
   <212> DNA
   <213> Artificial sequence
   <223> Primer
<400> 33
   TGTTCGTTGC TGGTGTTG 18
<210> 34
   <211> 20
   <212> DNA
   <213> Artificial sequence
   <223> Primer
<400> 34
   AACCTTCTCT TCCTACCCTT 20
<210> 35
   <211> 20
   <212> DNA
   <213> Artificial sequence
   <223> Primer
<400> 35
   TCAGCAATCA CGTACTCATC 20

## Description

### Technical Field

The present invention relates to biotechnology of plants. Particularly, the present invention relates to the gene cloning of *GS3,* a major QTL regulating grain weight and grain length, which is located in the pericentrometric region of chromosome 3 in rice.

### Background of Invention

Grain size of rice is an important economic trait because: (1) grain size is a major determinant of grain weight, which is one of the three components of grain yield, and therefore, grain size is an important trait for yield; (2) grain size is also an important trait of rice appearance because grain weight is positively correlated with several characters including grain length, grain width and grain thickness (Evans, 1972, Rice Breeding, Los Banos, International Rice Research Institute, Manila, pp. 499-511). In China, the USA and some Asian countries, *Indica* rice with long and slender grain is generally preferred by most consumers. In China, a length/width ratio of 2.8 is adopted as an enforced threshold for a national standard for high quality rice. Thus, understanding the genetic basis and molecular mechanisms of grain size is important in improving both rice yield and quality.

In addition, grain size also plays an important role in the study of evolution of cultivars. It is generally believed that wild type relatives are usually small and round in shape, and are thus favored under natural selection. After a long-term domestication and selection by humans, the shape of grain particles has changed significantly. Therefore, the study of the genetic basis of grain size provides clues for the study of the evolution of cultivars.

Grain size is atypical quantitative trait and is complex in its genetic basis. Utilization of molecular marker technology is able to separate and locate QTL (Quantitative Trait Loci) controlling quantitative traits, thus separating the complex quantitative traits to simple Mendelian factors for studies. By using aforesaid methods, many QTLs regulating rice grain size were identified in recent years. Among these QTLs, a major QTL (referred to as GS3 in the present invention) located in the pericentrometric region of rice chromosome 3 was detected by many researchers (Huang et al., 1997, Mol. Breed. 3:105-113; Redona and Mackill, 1998, Theor. Appl. Genet. 96:957-963; Kubo et al., 2001, Rice Genet. Newsl. 18:26-28; Thomson et al., 2003, Theor. Appl. Genet. 107:479-493; Aluko et al., 2004, Theor. Appl. Genet. 109:630-639). Using F_{2:3} and a recombinant inbred line population derived from a cross between Zhenshan 97 and Minghui 63, the present invention has detected a major QTL several times that is present in the *GS3* locus regulating both the grain length and the grain weight. This QTL explains over 55% of the total variation of grain length, as well as approximately 20% of the total variation of grain weight (Yu et al., 1997, Proc. Natl. Acad. Sci. USA 94:9226-9231; Li et al., 2000, Theor. Appl. Genet. 101:248-254; Tan et al., 2000, Theor. Appl. Genet. 101:823-829; Xing et al., 2001, Acta. Bot. Sin. 43:721-726; Xing et al., 2002, Theor. Appl. Genet. 105:248-257; Hua et al., 2002, Genetics 162:1885-1895). These results indicate that *GS3* gene can be stably expressed in various genetic backgrounds and different environments. Therefore, *GS3* gene is greatly potent and a good prospect for the improvement of both yield and quality traits of rice. The high resolution mapping of *GS3* gene and the cloning of the corresponding gene provide new genetic resources for the improvement of both yield and quality in rice breeding.

High resolution mapping of the quantitative traits in common population groups is very difficult because it is not easy to determine whether a major QTL or multiple minor QTL are detected (Yano et al., 1997, Plant Molecular Biology 35:145-153) in said population groups. Secondly, in said population groups, multiple QTLs affecting the same trait are separated, therefore the interference caused and the affects of the environmental factors greatly limit the resolution of the location of a QTL. Special population groups can be established from high resolution mapping of a QTL. A common approach is to establish a near isogenic line (NIL) of an interesting QTL and to eliminate most background differences besides the interesting QTL locus to make said QTL presented as typical Mendelian genetics. Said approach has played an important role in many high resolution mapping and gene cloning of QTLs. Li et al. mapped *GS3* to a region of 93.8-kb in length (Li et al., 2004, Genetics 168:2187-2195). Said results have provided a basis for the separation of mapping and the cloning of the *GS3* gene.

Upon aforesaid purposes, the present inventors have isolated and cloned a major gene *GS3* regulating grain weight and grain length in rice by the approach of mapping and cloning, therefore providing new genetic resources for the improvement of both yield and quality in rice breeding, and also providing clues for the study on the evolution of cultivars.

### Detailed Description of the Invention

The present invention relates to the isolation and cloning of the whole DNA fragment encoding a major gene regulating both grain weight and grain length in rice. The present invention also relates to the improvements in both yield and quality of rice using said gene. Said gene is named GS3. In particular the invention relates to
(1) an isolated polynucleotide comprising a nucleic acid sequence selected from
   (a) a polynucleotide comprising the nucleic acid sequence of SEQ ID NO: 2;
   (b) a polynucleotide encoding a polypeptide having the amino acid sequence of SEQ ID NO: 3;
   (c) a polynucleotide comprising a nucleic acid sequence complementary to the nucleic acid sequence of SEQ ID NO: 2;
   (d) a polynucleotide having at least 70% sequence identity to the polynucleotide of (a), (b) or (c), wherein the encoded polypeptide regulates grain weight and grain length in rice;
   (e) a polynucleotide encoding a polypeptide having at least 70% sequence identity to a polypeptide having the amino acid sequence of SEQ ID NO: 3, wherein the polypeptide regulates grain weight and grain length in rice; and
   (f) a polynucleotide comprising a promoter functional in a plant cell, operably joined to a coding sequence for a polypeptide having at least 70% sequence identity to a polypeptide having the amino acid sequence of SEQ ID NO: 3, wherein the encoded polypeptide regulates grain weight and grain length in rice;
(2) a recombinant DNA construct comprising a polynucleotide according to (1) above;
(3) a transformed cell or organism comprising a polynucleotide according to (1) above, and
(4) a substantially purified polypeptide comprising the amino acid sequence of SEQ ID NO: 3.

The invention established a near isogenic line (NIL) of *GS3. GS3* was finely mapped to a region of 7.9-kb in length using the approach of mapping and cloning. With the help of the sequence information of a whole cDNA in aforesaid region, the invention predicted and analyzed the gene structure of *GS3* and the protein encoded by *GS3.* It was found that *GS3* comprises 5 exons and encodes 232 amino acids. It was predicted based on bioinformatics that said protein contains conserved domains including a PEBP-like domain, a transmembrane domain, a cysteine-rich domain of TNFR (tumour necrosis factor receptor)/NGFR (nerve growth factor receptor) and a VWFC (von Willebrand factor type C) homologous domain. In addition, the inventors sequenced and compared three large grain species (Minghui 63, a *indica* rice in China; H94, from Shanghai Agrobiological Gene Center; 93-11, a rice variety that has been completely sequenced) and 3 small grain species (Zhenshan97, a *indica* rice in China; Chun 7, from Shanghai Agrobiological Gene Center; Niponbare, a rice variety that has been completely sequenced). It was found that there was only a single base variation in said region of 7.9-kb in length between large grain species and small grain species. Said variation was located in the end of the second exon of *GS3,* wherein a cysteine codon (TGC) in small grain species is mutated to the terminator codon TGA in large grain species. Said mutation causes *GS3* to be prematurely terminated in large grain species and leads to the loss of 178 amino acids, including part of the PEBP-like domain and three other conserved domains. It was found through bioinformatics analysis that the VWFC domain regulates growth and development signals in combination with the growth regulator TGF-β family members. In the large grain species, *GS3* lacks the VWFC domain, therefore it loses its capacity to regulate growth and development signaling, which finally leads to the change in grain length and grain weight.

The present invention has the following advantages:
(1) it is the first cloning of a gene that highly affects the grain length and grain weight in rice, therefore providing new gene resources for high yield and good quality in rice breeding, and providing a good technology example for the cloning of homologous genes in other species;
(2) the gene cloned in the present invention provides evidence for the study of domestication and molecular evolution of rice and other species.

### Description of Figures

Figure 1 shows the technical flowchart of the present invention;
Figure 2 shows the six cultivars used in sequence alignment;
Figure 3 shows the frequency distribution of 1,000-grain weight, grain length, grain width and grain thickness in the random BC₃ F₂ subpopulation; wherein
   (a) frequency distribution of 1,000-grain weight;
   (b) frequency distribution of grain length;
   (c) frequency distribution of grain width;
   (d) frequency distribution of grain thickness;
Figure 4 shows the map of the *GS3* locus on the molecular linkage map of chromosome 3 (unit in cM);
Figure 5 shows the maps of the gene region, wherein
   (a) The high-resolution map of the *GS3* gene. The numbers between molecular markers indicate the numbers of recombination events detected between the GS3 locus and respective markers. Genebank no. OSJNBa0030J19 is a BAC clone of Nipponbare encompassing the *GS3* locus;
   (b) Organization of the GS3 gene. The positions of exons (black boxes), 5' and 3' UTR (hatched boxes), translation start codon (ATG), translation stop codon (TGA), and one common single nucleotide mutation in the second exon between the two grain-length groups are indicated, in which a substitution of cysteine (TGC) in the small grain group mutated to the translation stop codon (TGA) in the large grain group;
   (c) Predicted sequence of the *GS3* gene expression product. The position of the amino acid change in large grain group (cysteine to stop codon) is indicated by an asterisk. The PEBP-like domain is indicated by dashed underline, the transmembrane region by single solid underline, the TNFR/NGFR family cysteine-rich domain by double underline, and the VWFC domain is boxed;

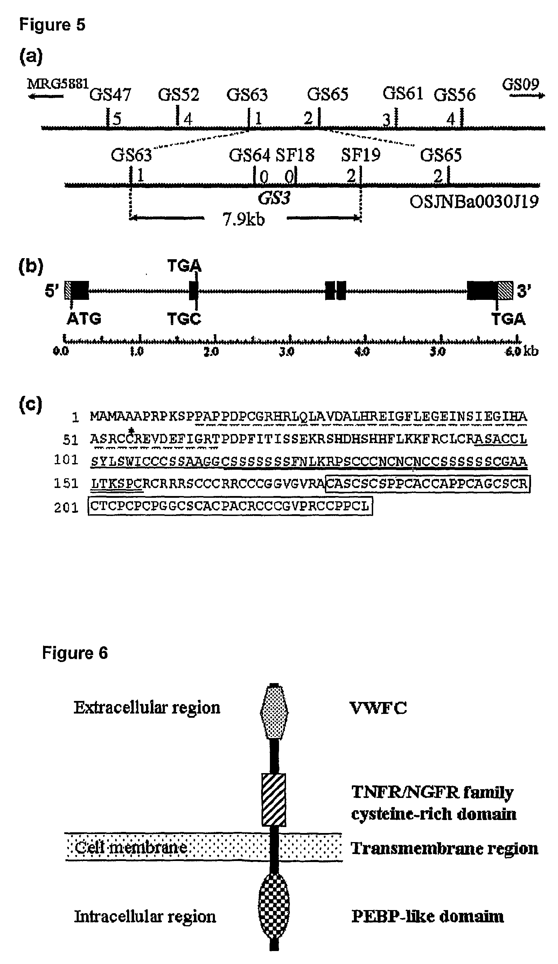

Figure 6 shows the organization of the predicted GS3 protein indicating the localization of the various conserved domains including the PEBP-like domain, the transmembrane domains, the cysteine-rich domain of TNFR/NGFR and the VWFC homologous domain, wherein the PEBP-like domain is located inside the membrane, while the cysteine-rich domain of TNFR and the VWFC homologous domain are located outside the membrane.

### Examples

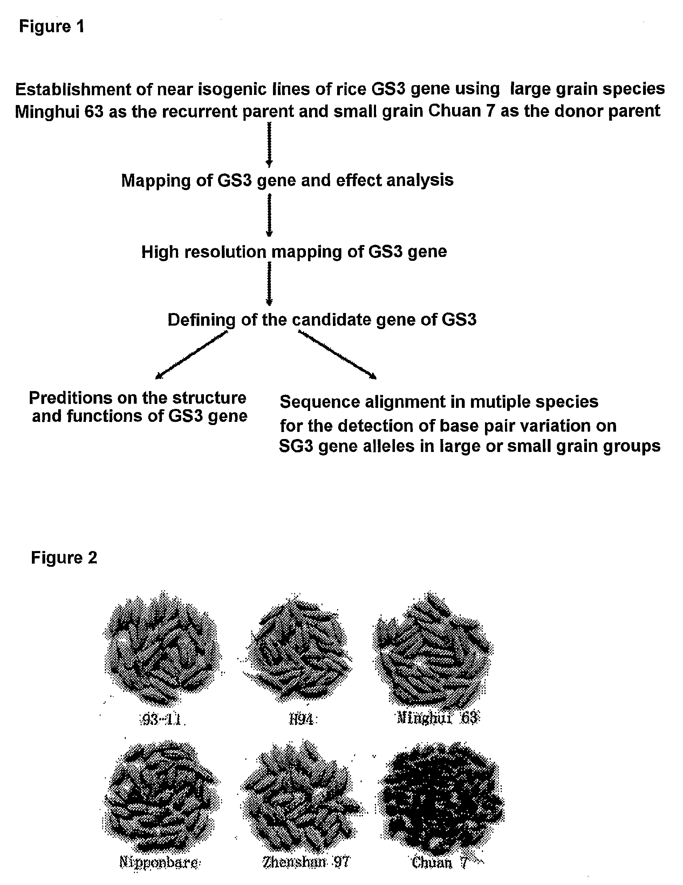

A near isogenic line of rice *GS3* gene was established using Minghui 63 as the recurrent parent and Chuan 7 as the donor parent. Mapping and effect evaluation of *GS3* gene were carried out on a random BC₃ F₂ subpopulation consisting of 201 BC₃F₂ individuals. By further analysis on 1,384 BC₃F₂ individuals with large grain phenotype using CAPS markers, *GS3* was finally mapped between the two CAPS markers GS63 and SF19 (designed by the inventors; see Table 2), which was approximately 7.9kb in distance. Based on a whole length cDNA sequence, the *GS3* gene structure was predicted. In addition, the possible function of *GS3* gene was predicted based on the bioinformational technology. It was found by sequence alignment of the 7.9kb fragment that a mutation was presented in all the large grain species which led to deficiency of 178 amino acids. Such deficiency could lead to the loss of the gene function. It implied the essential reason of the change in grain size, and at the same time, proved the correctness of setting *GS3* as the target gene (Figure 1).

The following Examples further define the present invention and describe the methods for the isolation and cloning of *GS3* gene, as well as the methods for the detection of the base mutation between *GS3* alleles by sequence alignment.

### Example 1: establishment of a near isogenic lines of rice GS3 gene

### 1. Backcrossing and Screening

As shown in Figure 2, successive crossing was carried out using Minghui 63 (large grain) as the recurrent parent and Chuan 7 (small grain) as the donor parent. Positive selection of *GS3* was carried out in F₁, BC₁F₁ and BC₂F₁, that is, selecting individual plants whose targeting region was Minghui63/Chuan7 heterozygous genotype for the following backcrossing. The targeting region was determined in a region between two known SSR (Simple Sequence Repeat) markers RM282 and RM16. In BC₃F₁, in addition to positive selection, surveillance was also carried out in the genetic backgrounds besides the targeting region. Individual plants with a genetic background closest to Minghui63 were selected for the following experiments. Referring to the published rice genetic linkage map (Temnykh et al., 2000, Theor. Appl. Genet. 100:697-712; Temnykh et al., 2001, Genome Res. 11:1441-1452), 125 SSR markers with known polymorphism in the parents and evenly distributed on the 12 rice chromosomes were selected for surveillance of genetic backgrounds. One plant (BC₃F₁-19) was finally selected, whose genotype in the RM282 and RM16 region was Minghui63/Chuan7 heterozygous genotype, while whose genotype in said 125 SSR markers, only about 20% (25 pairs) of the markers were Minghui63/Chuan7 heterozygous genotype, and the rest thereof were all Mingui63 homozygous genotype. The progeny (BC₃F₂ and BC₃F₃) of said individual plant were used in the following experiments.

### 2. SSR methods

The standard PCR protocol followed the methods taught in Sambrook, J. et al., Molecular Cloning: A Laboratory Manual, 3rd ed., Translated by Jin Dong Yan et al., Science Press. In the PCR, a 20µl reaction system was used, which contained: 20-50ng DNA template, 10 mM Tris-HCl, 50 mM KCl, 0.1% Triton X-100, 1.8 mM MgCl₂, 0.1 mM dNTP, 0.2 µM primers (primers of RM282 and RM16 as mentioned above) and 1 U Taq DNA polymerase. Conditions for PCR included: 94°C predenature for 4 min; 94°C 1min, 55°C 1min, 72°C1min, 34 cycles; 72°C elongation 10min. PCR products were separated on a 6% acrylamide gel and then silver-stained (Bassam et al., 1991, Anal. Biochem. 196: 80-83).

### Example 2: Mapping and effect evaluation of GS3 in the random subpopulation

### 1. Measurements of traits of large and small grain

Grain particles were air-dried and stored at room temperature for at least 3 months before testing in order to make sure the dryness and water contents thereof were relatively identical. Ten randomly chosen full filled grains from each plant were lined up closely in a way that each lay head to head, tail to tail, with no overlap and no gap in between. Said grains were arranged length-wise to measure the grain length using a vernier caliper, and then were lined up closely side by side, that is, arranged by breadth to measure the grain width using a vernier caliper. Grain thickness was determined for each grain individually using a vernier caliper, and the values were averaged and used as the measurements for the plant. Grain weight was calculated based on 200 randomly chosen fully filled grains and converted to 1,000-grain weight.

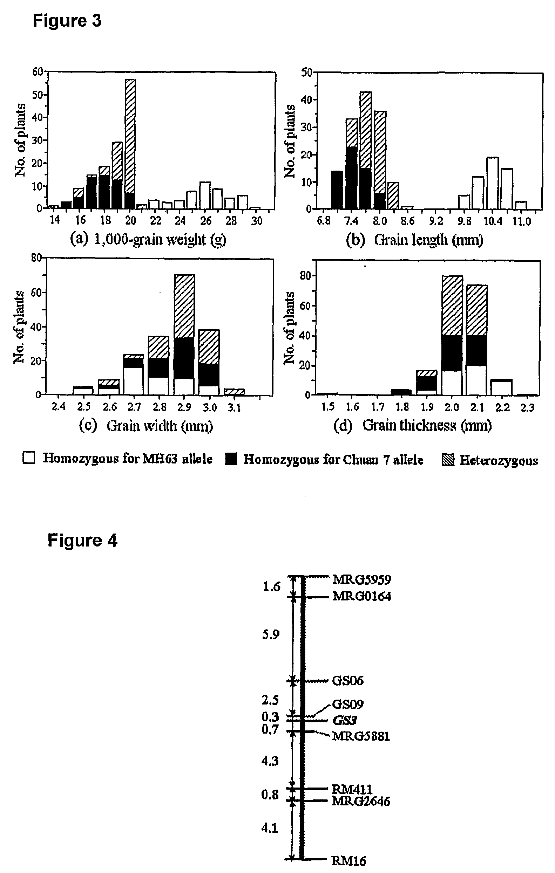

201 individuals of BC₃F₂ derived from the BC₃F₁-19 individual plant were randomly selected to form a random subpopulation. Distributions of grain weight, grain length, grain width and grain thickness were studied in Minhui 63, Chuan 7 and said random subpopulation of BC₃F₂. It was found that all the traits were significantly different between the two parents (Table 1). In the random subpopulation, both grain length and 1,000-grain weight expressed a discontinuous distribution. The plants were classified as long grain and short grain based on a boundary of 8.50-9.50 mm in grain length or 20.5-21.5g in 1,000-grain weight (Table 1 and Figure 3). Grain length concurred completely with grain weight, such that long grains were heavier than short grains, and vice visa. However, grain width and thickness showed normal distributions (Figure 3). For simplicity, in the present invention the large and heavier grains are referred to as long grain, and the opposite type as short grain.

**Table 1. Descriptive statistics of the traits for the two parents and the long grains and short grains in the random subpopulation**

| Trait | Parent (mean ± SD) | | MM | | CC | | MC | |
|---|---|---|---|---|---|---|---|---|
| | Minghui 63 | Chuan 7 | Mean ± SD | Range | Mean ± SD | Range | Mcan ± SD | Range |
| 1.000-grain weight (g) | 28.6±0.6 | 12.5±0.4 | 25.6±2.0 | 21.5-29.8 | 17.5±1.3 | 14.2-20.0 | 19.0±1.2 | 14.0-20.5 |
| Grain length (mm) | 9.91±0.099 | 6.30±0.089 | 10.25±0.29 | 9.64-10.73 | 7.32±0.26 | 6.86-7.84 | 7.72±0.25 | 7.24-8.50 |
| Grain width (mm) | 2.80±0.03 | 2.48±0.02 | 2.72±0.13 | 2.43-2.96 | 2.82±0.12 | 2.45-3.06 | 2.85±0.09 | 2.56-3.04 |
| Grain thickness (mm) | 2.13±0.06 | 1.68±0.03 | 2.03±0.09 | 1.81-2.21 | 1.95±0.10 | 1.45-2.10 | 1.99±0.06 | 1.79-2.13 |

### 2. Design of molecular markers

Some SSR markers used in the present invention are publicly known. In addition, 11 pairs of Indel (Insert/Deletion) and CAPS (cleaved amplified polymorphic sequence) markers which shows polymorphism between Minghui 63 and Chuan 7 were designed based on the genome DNA sequence of Japonica Rice, Nipponbare and India Rice, 93-11. Said Indel and CAPS markers were used in the high resolution mapping analysis of *GS3.* The DNA sequences of said markers are listed in Table 2.

**Table 2. Indel and CAPS markers (primers) developed for the mapping of the GS3 gene locus**

| Marker | Type | Forward primer (5'-3') | Reverse primer (5'-3') | Annealing Temp.(°C) | Restriction enzyme |
|---|---|---|---|---|---|
| GS06 | Indel | AGCAAAGCTGGAACGAAGAG (SEQ ID NO. 4) | TAAATTACGCCGTGTCAACG (SEQ ID NO. 5) | 55 | |
| GS09 | Indel | GCAACCAAGTCCACGCTAAT (SEQ ID NO. 6) | TAGCCGAAGATCAGCCTCCT (SEQ ID NO. 7) | 57 | |
| GS47 | CAPS | GATTATTGGAGACGGGACGA (SEQ ID NO. 8) | GACGGCATGACCACTCTTTT (SEQ ID NO. 9) | 55 | HapII |
| GS52 | CAPS | AGCTTTGGTGTCGTTCTGCT (SEQ ID NO. 10) | CCGACTTGGAGAGAATGGAA (SEQ ID NO. 11) | 55 | BglI |
| GS56 | CAPS | GCTGTGTTGTCCTTTGCTGA (SEQ ID NO. 12) | CCAATAAACCCCACTGCAAC (SEQ ID NO. 13) | 55 | BglI |
| GS61 | CAPS | CTTTACAAAACCGGCGGTAA (SEQ ID NO. 14) | TGAAGCGGACCTAGCATTTT (SEQ ID NO. 15) | 53 | BelI |
| GS63 | CAPS | AAGAACGACTACGCGCATCT (SEQ ID NO. 16) | CCATCGCTCTCTTTCCTCAG (SEQ ID NO. 17) | 53 | HhaI |
| GS64 | CAPS | CAACACCAGCAACGAACAAC (SEQ ID NO. 18) | ACGAGGGATTATCAGCCATT (SEQ ID NO. 19) | 55 | EcoRI, HapII |
| GS65 | CAPS | CGGTATGCCAAGTTGAATGA (SEQ ID NO. 20) | TTGCCGCAGTAAACAAGAAG (SEQ ID NO. 21) | 55 | HhaI, HapII |
| SF18 | CAPS | CCTTCAGTAAGAGAGATGTG (SEQ ID NO. 22) | AGTTGATGGTTTTGTGGGAT (SEQ ID NO. 23) | 57 | BclI |
| SF19 | CAPS | TCTGCTTGCGGTTATCTGTA (SEQ ID NO. 24) | TTAGGTCCCTTTTCTCGTCC (SEQ ID NO. 25) | 57 | SacI |

| | | | | | |
|---|---|---|---|---|---|
| Remark: Markers (primers) GS63 and SF19 were designed by the inventors. | | | | | |

### 3. QTL mapping and effect evaluation of GS3

Said random subpopulation was subjected to genotype analysis using 6 SSR markers (MRG5959, MRG0164, MRG5881, MRG2646, RM411, RM16) and 2 Indel markers (GS06 and GS09). Mapmaker/Exp 3.0 (Lincoln et al.1992, Whitehead Institute Technical Report, Whitehead Institute, Cambridge, Massachusetts, USA) was used to establish a partial genetic linkage map in *GS3* region. QTL analysis on the traits including grain length, width, thickness and 1,000-grain weight of the random subpopulation was conducted using the program Mapmaker/QTL 1.1 at a threshold of LOD 3.0. QTL analysis indicated that a QTL found in the interval between GS09 and MRG5881 had effects simultaneously on grain weight, grain length, grain width and grain thickness, and contributed 83.4%, 95.6%, 19.8% and 12.1% of the phenotypic variation on these traits, respectively. The allele from Minghui 63 contributed to the increase of grain weight, grain length and grain thickness, but to the decrease of grain width. Moreover, the QTL also showed different modes of gene actions on the traits, such that partial dominance was observed for 1000-grain weight, grain length and grain thickness, while overdominance was detected for grain width.

**Table 3. Effects of the QTL (in the interval GS09-MRG5881) on grain shape and weight**

| Traits | LOD | A^{a} | D^{b} | Var. %^{c} |
|---|---|---|---|---|
| 1,000-grain weight (g) | 72.8 | -4.08^{d} | -2.52^{d} | 83.4 |
| Grain length (mm) | 129.2 | -1.47^{d} | -1.06^{d} | 95.6 |
| Grain width (mm) | 8.9 | 0.05^{d} | 0.08^{d} | 19.8 |
| Grain thickness (mm) | 5.3 | -0.04^{d} | 0.004^{e} | 12.1 |

| | | | | |
|---|---|---|---|---|
| ^{a}. Additive effect of Chuan 7 allele ^{b}. Dominance effect of Chuan 7 allele ^{c}. Percentage of total phenotypic variance explained by the QTL ^{d}. Significant at P <0.0001 in t test ^{e}. Not significant at P <0.05 | | | | |

### 4. Progeny test

Each plant in said random subpopulation was bred to 20 families (BC₃ F₃) which were subjected to progeny test. Progenies of 56 in 201 families were uniformly long grains, while 61 families were uniformly short grains and 84 families had both long and short grains. The ratio of the three groups fit well to the expected ratio (1:2:1) of single locus Mendelian segregation (χ2=5.67,P >0.05) in the χ2 test. The results indicated that in this BC₃ F₂ subpopulation, the grain size was controlled by a major gene, and the small size allele is dominant over the large size allele. The three distinct phenotypic classes corresponded to the three genotypes of the BC₃ F₂ individuals at the GS3 locus: homozygote for the Minghui 63 allele (long grain), homozygote for the Chuan 7 alleles (short grain), and heterozygote. Using the three phenotypic classes as a marker, *GS3* was directly mapped into a 1-cM region delimitated by an Indel marker GS09 and SSR marker MRG5881 (Figure 4).

### Example 3. High resolution mapping of GS3

### 1. CAPS analysis

9 CAPS markers used for the high resolution locating are listed in Table 2. The amplification product amplified by said markers had a size of around 1-kb. The PCR reaction system was identical with the SSR reaction system mentioned above. The amplification condition of the PCR was as below: 94°C predenature 4min; 94°C 1min, 53°C∼57°C 1min, 72°C 1.5min, 34 cycles; 72°C elongation 10min. The digestion of the amplicons was carried out in a 20µl reaction system containing: 10µl PCT product, 1 U restriction digestive enzyme (from Takara Ltd., Japan). Additional components were as described in the manual provided by Takara Ltd. After digestion in 37°C for 3-5 hours, 10µl of the digestion product was subjected to separation by electrophoresis in a 1.5% agrose gel, which was then observed using UV after EB staining.

### 2. Analysis of the recombinant plant and high resolution mapping of GS3

To further narrow down the GS3 containing genomic region, 1,384 plants with long grain phenotype (long grain, 9.7 mm or longer) from the BC₃ F₂ population of 5,740 individuals derived from BC₃F₁-19 individual plant were selected for recombinant screening.

All of the 1,384 selected plants were screened using an SSR marker MRG5881 and Indel marker GS09, which identified a total of 55 recombinants which were further confirmed to be very large size singles by progeny test. Using 9 designed CAPS markers to screen the 55 recombinants, it was found that five recombination events were resolved between GS47 and GS3, four identified between GS52 and GS3, four between GS56 and GS3, three between GS61 and GS3, and two between GS65 and GS3 (Figure 5a). In particular, the assay revealed one recombination event between GS63 and GS3 and two recombination events between SF19 and GS3. In addition, GS64 and SF 18 were found to co-segregate with the GS3 locus. Therefore, the genomic region containing the GS3 locus was narrowed down to the DNA fragment bounded by GS63 and SF19, which corresponded to approximately 7.9-kb in length in the genome sequence ofNipponbare and 93-11(Figure 5a).

### Example 4. Gene structure and predicted function analysis of GS3

### (1) Gene structure analysis of GS3

A full-length cDNA, which is named osigcea013f09t3, from the plumule of an indica cultivar Guangluai 4 (provided by Shanghai Agrobiological Gene Center) was identified in the 7.9-kb fragment (Fig.4b) between GS63 and SF19 (which were designed by the applicants; refer to Table 2). The nucleotide sequence is shown in SEQ ID NO: 1. The cDNA sequence of the cloned *GS3* gene of the present invention is 953bp in length, which matches well with the region between positions 1.6 and 7.3 kb of the 7.9-kb fragment. Allowing for the regulatory regions on both ends, this is considered as the only candidate gene for *GS3.*

The structure of *GS3* gene was obtained by comparing the sequences of said total cDNA with the genomic DNA sequence of Nipponbare. *GS3* gene is 5,363bp in length from the translation start codon to the termination codon. It comprises 5 exons and 4 introns. The starting exon is 117bp in length, while the second exon is 53bp, third exon 45bp, fourth exon 54bp, terminal exon 430bp in length, respectively. The first intron is 1,472bp in length, while the second intron is 1,439bp, third intron 83bp, fourth intron 1,671bp in length, respectively (Table 5b). Therefore, the open reading frame of *GS3* gene is 699bp in length and encodes 232 amino acids. The sequence of said gene is shown in SEQ ID NO:1.

### (2) Function prediction of GS3

Prediction for the structure of GS3 protein was carried out with InterProScan. It was revealed that the protein encoded by *GS3* gene consists of 232 amino acids and comprises several conserved domains. There is a PEBP (phosphatidylethanolamine-binding protein)-like domain in amino acid 12-65 at the 5' terminus. A transmembrane region is located at amino acid 97-117. The region of amino acid 116-1557 is a TNFR (tumor necrosis factor receptor)/NGFR (nerve growth factor receptor) family cysteine-rich domain. The 3' terminal cysteine-rich region shows the characters of the conserved amino acid sequence of the von Willebrand factor type C (VWFC) domain, which is typically 60 -80 amino acid in length and comprises ten cysteines, especially is characterized in that it contains a C2XXC3XC4 sequence located in the middle and a C8C9XXC10 sequence at the 3' terminal end (wherein C represents cysteine; the number represents the order of said conserved cysteines; X indicates any amino acid) (Figure 5c and Figure 6).

VWFC domain is represented in a number of extracellular matrix proteins. Some studies show that VWFC binds to members of the transforming growth factor TGF-β superfamily, thus disrupting the receptor binding sites of TGF-b superfamily proteins and preventing activation of the TGF-b receptor, such that it regulates the growth factor signaling pathway (Abreu et al., 2002, Gene 287:39-47; O'Leary et al., 2004, J. Biol. Chem. 279:53857-53866).

### Example 5: Detection of base pair variation between GS3 alleles by sequence alignment

### (1) Sequencing

Two large grain species (Minghui 63 and H94) and two small grain species (Chuan 7 and Zhenshan 97) were sequenced in the target genomic DNA region. DNA fragments from these cultivars were amplified using 10 pairs of primers whose amplicons were partially overlaping with each other. The amplification was carried out with high fidelity LA-Taq (TakaRa, Dalian, China). The PCR products were cloned into pGEM-T vector (Promega, USA) according to the manufacturer's specification. The cloned product was transformed into *E. coli* DH10B (Invitrogen, USA) and positive clones were screened with blue-white methods. The T7-R and SP6-F universal primers (Shanghai Sangon Biological Engineering Technology and Services Co. LTD.) and the Big Dye Terminator Cycle Sequencing v3.1 (Applied Biosystems, Foster City, CA, USA) were used for sequencing from both ends of the subclones. Sequence contigs were assembled using the computer program SEQUENCHER 4.1 (Gene Codes Corporation, USA).

**Table 4. Primers used in the sequence alignment.**

| Marker | Forward primer (5'-3') | Reverse primer (5'-3') | Size of Amplicon | Annealing Temp.(°C) |
|---|---|---|---|---|
| GS63 | AAGAACGACTACGCGCATCT (SEQ ID NO. 16) | CCATCGCTCTCTTTCCTCAG (SEQ ID NO. 17) | 704bp | 53 |
| SF26 | GTCTGAGGAAAGAGAGCGAT (SEQ ID NO. 26) | AAGCAAGCCAAGGGAAATGT (SEQ ID NO. 27) | 1091bp | 60 |
| SF15 | AGCAAAAAAGGTGAAGGACG (SEQ ID NO. 28) | CAAAGGGAATAACAAGGCAG (SEQ ID NO. 29) | 1295bp | 57 |
| SF16 | CGAATAGGAAGTCAATGGC (SEQ ID NO. 30) | GTCGTACCCGCCTTAGTTGA (SEQ ID NO. 31) | 1159bp | 55 |
| SF28 | TGCCCATCTCCCTCGTTTAC (SEQ ID NO. 32) | TGTTCGTTGCTGGTGTTG (SEQ ID NO.33) | 1065bp | 55 |
| GS64 | CAACACCAGCAACGAACAAC (SEQ ID NO. 18) | ACGAGGGATTATCAGCCATT (SEQ ID NO. 19) | 1155 bp | 55 |
| SF18 | CCTTCAGTAAGAGAGATGTG (SEQ ID NO. 22) | AGTTGATGGTTTTGTGGGAT (SEQ ID NO. 23) | 1245 bp | 57 |
| SF45 | AACCTTCTCTTCCTACCCTT (SEQ ID NO. 34) | TCAGCAATCACGTACTCATC (SEQ ID NO. 35) | 1137 bp | 55 |
| SF19 | TCTGCTTGCGGTTATCTGTA (SEQ ID NO. 24) | TTAGGTCCCTTTTCTCGTCC (SEQ ID NO. 25) | 1224 bp | 57 |

The size of the PCR products depended on the sequence of Nipponbare and varied among different species.

### (2) Sequence alignment

Sequence alignment was carried out on three large grain varieties (Minghui 63, H94, 93-11) and three small grain varieties (Zhenshan97, Chun 7, Niponbare) (Figure 2). The *GS3* region sequences of Nipponbare and 93-11 were from the BAC clone OSJNBa0030J19 and contig Ctg009226, respectively. Sequence alignment was conducted using the computer program Vector NTI 9 (InforMaxTM Corporation, USA).

Although many nucleotide changes were observed among the six cultivars in the 7.9-kb region, there was only one common single nucleotide mutation detected between these two different grain-length groups, which indicated said mutation was the essential reason causing the grain size change. Further studies showed that said nucleotide mutation was located at the second exon of the *GS3* gene, in which a cysteine codon (TGC) in the small-grain group was mutated to a termination codon (TGA) in the large-grain group (Fig.4b). This premature termination resulted in a 178-amino acids truncation in the C-terminus of the predicted protein in the large-grain group, which eliminated part of the PEBP-like domain and all the other three conserved domains. Such nonsense mutation was clearly in agreement with the recessive nature of the long grain phenotype, indicating that long grains resulted from the loss of the function of the protein otherwise producing short grains.

## Claims

1. An isolated polynucleotide comprising a nucleic acid sequence selected from
(a) a polynucleotide comprising the nucleic acid sequence of SEQ ID NO: 2;
(b) a polynucleotide encoding a polypeptide having the amino acid sequence of SEQ ID NO: 3;
(c) a polynucleotide comprising a nucleic acid sequence complementary to the nucleic acid sequence of SEQ ID NO: 2;
(d) a polynucleotide having at least 70% sequence identity to the polynucleotide of (a), (b) or (c), wherein the encoded polypeptide regulates grain weight and grain length in rice;
(e) a polynucleotide encoding a polypeptide having at least 70% sequence identity to a polypeptide having the amino acid sequence of SEQ ID NO: 3, wherein the polypeptide regulates grain weight and grain length in rice; and
(f) a polynucleotide comprising a promoter functional in a plant cell, operably joined to a coding sequence for a polypeptide having at least 70% sequence identity to a polypeptide having the amino acid sequence of SEQ ID NO: 3, wherein the encoded polypeptide regulates grain weight and grain length in rice.

2. The isolated polynucleotide according to claim 1 which is a polynucleotide comprising the nucleic acid sequence of SEQ ID NO: 2 or a complement thereof.

3. The isolated polynucleotide according to claim 1, which encodes a polypeptide having the amino acid sequence of SEQ ID NO: 3.

4. The isolated polynucleotide according to claim 1, which encodes a polypeptide having at least 70% amino acid sequence identity with a polypeptide having the amino acid sequence of SEQ ID NO: 3, wherein the polypeptide regulates grain weight and grain length in rice.

5. A recombinant DNA construct comprising a polynucleotide according to claim 1 or 2.

6. The recombinant DNA construct according to claim 5, wherein the polynucleotide is operably linked to a promoter functional in a plant cell.

7. The recombinant DNA construct according to claim 5, wherein the polynucleotide is operably linked to a 3' untranslated region functional in a plant cell.

8. A transformed cell or organism comprising a polynucleotide according to claim 1 or 2.

9. The transformed cell or organism according to claim 8, wherein the cell is a plant cell or plant.

10. The transformed cell or organism according to claim 9, wherein the organism is a plant selected from cotton, wheat, soybean, maize, rice, and canola.

11. The transformed cell or organism according to claim 8, which is a transformed plant comprising a recombinant DNA construct, wherein the construct comprises a promoter region functional in a plant cell operably joined to a polynucleotide comprising a coding sequence for a polypeptide having the amino acid sequence of SEQ ID NO: 3.

12. A transformed cell or organism of claim 11 wherein the polynucleotide is oriented with respect to the promoter such that transcription of the polynucleotide produces an mRNA encoding the polypeptide.

13. A transformed cell or organism of claim 11 wherein the polynucleotide is oriented with respect to the promoter such that transcription from the polynucleotide produces an RNA complementary to the mRNA encoding the polypeptide.

14. The transformed cell or organism according to claim 12, wherein the plant is selected from cotton, wheat, soybean, maize, rice, and canola.

15. A substantially purified polypeptide comprising the amino acid sequence of SEQ ID NO: 3.

## Patentansprüche

1. Isoliertes Polynucleotid, das eine Nucleinsäuresequenz umfasst, die ausgewählt ist aus
(a) einem Polynucleotid, das die Nucleinsäuresequenz von SEQ ID Nr. 2 umfasst;
(b) einem Polynucleotid, das ein Polypeptid codiert, welches die Aminosäuresequenz von SEQ ID Nr. 3 aufweist;
(c) einem Polynucleotid, das eine Nucleinsäuresequenz umfasst, die zu der Nucleinsäuresequenz von SEQ ID Nr. 2 komplementär ist;
(d) einem Polynucleotid, das wenigstens 70% Sequenzidentität zu dem Polynucleotid von (a), (b) oder (c) aufweist, wobei das codierte Polypeptid das Korngewicht und die Kornlänge bei Reis reguliert;
(e) einem Polynucleotid, das ein Polypeptid codiert, welches wenigstens 70% Sequenzidentität zu einem Polypeptid aufweist, das die Aminosäuresequenz von SEQ ID Nr. 3 aufweist, wobei das Polypeptid das Korngewicht und die Kornlänge bei Reis reguliert; und
(f) einem Polynucleotid, das einen Promotor umfasst, der in einer Pflanzenzelle funktionsfähig ist und der funktionell mit einer codierenden Sequenz für ein Polypeptid verknüpft ist, das wenigstens 70% Sequenzidentität zu einem Polypeptid aufweist, welches die Aminosäuresequenz von SEQ ID Nr. 3 aufweist, wobei das codierte Polypeptid das Korngewicht und die Kornlänge bei Reis reguliert.

2. Isoliertes Polynucleotid gemäß Anspruch 1, bei dem es sich um ein Polynucleotid handelt, das die Nucleinsäuresequenz von SEQ ID Nr. 2 oder ein Komplement davon umfasst.

3. Isoliertes Polynucleotid gemäß Anspruch 1, das ein Polypeptid codiert, welches die Aminosäuresequenz von SEQ ID Nr. 3 aufweist.

4. Isoliertes Polynucleotid gemäß Anspruch 1, das ein Polypeptid codiert, welches wenigstens 70% Aminosäuresequenzidentität zu einem Polypeptid aufweist, das die Aminosäuresequenz von SEQ ID Nr. 3 aufweist, wobei das Polypeptid das Korngewicht und die Kornlänge bei Reis reguliert.

5. Rekombinantes DNA-Konstrukt, das ein Polynucleotid gemäß Anspruch 1 oder 2 umfasst.

6. Rekombinantes DNA-Konstrukt gemäß Anspruch 5, wobei das Polynucleotid funktionell mit einem in einer Pflanzenzelle funktionsfähigen Promotor verknüpft ist.

7. Rekombinantes DNA-Konstrukt gemäß Anspruch 5, wobei das Polynucleotid funktionell mit einem in einer Pflanzenzelle funktionsfähigen 3'-untranslatierten Bereich verknüpft ist.

8. Transformierte Zelle oder transformierter Organismus, umfassend ein Polynucleotid gemäß Anspruch 1 oder 2.

9. Transformierte Zelle oder transformierter Organismus gemäß Anspruch 8, wobei die Zelle eine Pflanzenzelle oder Pflanze ist.

10. Transformierte Zelle oder transformierter Organismus gemäß Anspruch 9, wobei der Organismus eine Pflanze ist, die aus Baumwolle, Weizen, Soja, Mais, Reis und Canola ausgewählt ist.

11. Transformierte Zelle oder transformierter Organismus gemäß Anspruch 8, bei dem es sich um eine transformierte Pflanze handelt, die ein rekombinantes DNA-Konstrukt umfasst, wobei das Konstrukt einen Promotorbereich umfasst, der in einer Pflanzenzelle funktionsfähig ist und der funktionell mit einem Polynucleotid verknüpft ist, das eine codierende Sequenz für ein Polypeptid umfasst, welches die Aminosäuresequenz von SEQ ID Nr. 3 aufweist.

12. Transformierte Zelle oder transformierter Organismus gemäß Anspruch 11, wobei das Polynucleotid in Bezug auf den Promotor so orientiert ist, dass die Transcription des Polynucleotids eine mRNA erzeugt, die das Polypeptid codiert.

13. Transformierte Zelle oder transformierter Organismus gemäß Anspruch 11, wobei das Polynucleotid in Bezug auf den Promotor so orientiert ist, dass die vom Polynucleotid ausgehende Transcription eine RNA erzeugt, die zu der das Polypeptid codierenden mRNA komplementär ist.

14. Transformierte Zelle oder transformierter Organismus gemäß Anspruch 12, wobei die Pflanze aus Baumwolle, Weizen, Soja, Mais, Reis und Canola ausgewählt ist.

15. Im Wesentlichen gereinigtes Polypeptid, das die Aminosäuresequenz von SEQ ID Nr. 3 umfasst.

## Revendications

1. Polynucléotide isolé comprenant une séquence d'acide nucléique choisie parmi
(a) un polynucléotide comprenant la séquence d'acide nucléique de SEQ ID No : 2 ;
(b) un polynucléotide codant pour un polypeptide ayant la séquence d'acides aminés de SEQ ID No : 3 ;
(c) un polynucléotide comprenant une séquence d'acide nucléique complémentaire de la séquence d'acide nucléique de SEQ ID No : 2 ;
(d) un polynucléotide présentant une identité de séquence d'au moins 70 % avec le polynucléotide (a), (b), ou (c), dans lequel le polypeptide codé régule le poids de grain et la longueur de grain du riz ;
(e) un polynucléotide codant pour un polypeptide présentant une identité de séquence d'au moins 70 % avec un polypeptide ayant la séquence d'acides aminés de SEQ ID No : 3, dans lequel le polypeptide régule le poids de grain et la longueur de grain du riz ; et
(f) un polynucléotide comprenant un promoteur opérationnel dans une cellule végétale, fonctionnellement lié à une séquence codant pour un polypeptide présentant une identité de séquence d'au moins 70 % avec un polypeptide ayant la séquence d'acides aminés de SEQ ID No : 3, dans lequel le polypeptide codé régule le poids de grain et la longueur de grain du riz.

2. Polynucléotide isolé selon la revendication 1 qui est un polynucléotide comprenant la séquence d'acide nucléique de SEQ ID No : 2 ou un complément de celle-ci.

3. Polynucléotide isolé selon la revendication 1, qui code pour un polypeptide ayant la séquence d'acides aminés de SEQ ID No : 3.

4. Polynucléotide isolé selon la revendication 1, qui code pour un polypeptide présentant une identité de séquence d'acides aminés d'au moins 70 % avec un polypeptide ayant la séquence d'acides aminés de SEQ ID No : 3, dans lequel le polypeptide régule le poids de grain et la longueur de grain du riz.

5. Construction d'ADN recombiné comprenant un polynucléotide selon la revendication 1 ou 2.

6. Construction d'ADN recombiné selon la revendication 5, dans laquelle le polynucléotide est fonctionnellement lié à un promoteur opérationnel dans une cellule végétale.

7. Construction d'ADN recombiné selon la revendication 5, dans laquelle le polynucléotide est fonctionnellement lié à une région 3' non traduite opérationnelle dans une cellule végétale.

8. Cellule ou organisme transformé comprenant un polynucléotide selon la revendication 1 ou 2.

9. Cellule ou organisme transformé selon la revendication 8, dans lequel la cellule est une cellule végétale ou une plante.

10. Cellule ou organisme transformé selon la revendication 9, dans lequel l'organisme est une plante choisie parmi le coton, le blé, le soja, le maïs, le riz, et le colza.

11. Cellule ou organisme transformé selon la revendication 8, qui est une plante transformée comprenant une construction d'ADN recombiné, dans lequel la construction comprend un promoteur opérationnel dans une cellule végétale, fonctionnellement lié à un polynucléotide comprenant une séquence codant pour un polypeptide ayant la séquence d'acides aminés de SEQ ID No : 3.

12. Cellule ou organisme transformé selon la revendication 11, dans lequel le polynucléotide est orienté par rapport au promoteur de façon que la transcription du polynucléotide produise un ARNm codant pour le polypeptide.

13. Cellule ou organisme transformé selon la revendication 11, dans lequel le polynucléotide est orienté par rapport au promoteur de façon que la transcription du polynucléotide produise un ARN complémentaire de l'ARNm codant pour le polypeptide.

14. Cellule ou organisme transformé selon la revendication 12, dans lequel la plante est choisie parmi le coton, le blé, le soja, le maïs, le riz, et le colza.

15. Polypeptide substantiellement purifié comprenant la séquence d'acides aminés de SEQ ID No : 3.
